# DEMANDE DE BREVET EUROPEEN

(11) **EP 4 029 534 A1**
(43) Date de publication de la demande: **20.07.2022**
(21) Numéro de dépôt: 21152105.9
(22) Date de dépôt: 18.01.2021
(51) Int. Cl.: A61L 9/013, A61L 9/00

(54) **DISPOSITIF DE PURIFICATION D'AIR**

(71) Demandeur: FläktGroup France SAS, 59223 Roncq (FR)
(72) Inventeur: TELLE, Sébastien, 59223 Roncq (FR)
(74) Mandataire: Cabinet Netter

(57) **Abrégé**

[Dispositif de purification d'air 1, comprenant un châssis 3 déplaçable, un organe d'amenée 23 d'air ambiant dans le dispositif, un filtre actif 21, un agent actif biodécontaminant déposé à la surface du filtre actif 21, l'organe d'amenée d'air étant disposé en aval ou en aval du filtre actif 21 dans le sens de circulation de l'air.]

## Description

La présente invention concerne le domaine de la purification d'air.

On connaît des filtres installés dans des systèmes de ventilation. Les filtres sont changés à intervalles réguliers. Le changement de filtre expose l'opérateur à des pathogènes arrêtés par le filtre. Ceci est particulièrement sensible pour des pathogènes bactériens ou viraux.

L'invention vient y remédier.

L'invention propose un dispositif de purification d'air, comprenant un châssis déplaçable, un organe d'amenée d'air ambiant dans le dispositif, un filtre actif, un agent actif biodécontaminant déposé à la surface du filtre actif, l'organe d'amenée d'air étant disposé en aval ou en amont du filtre actif dans le sens de circulation de l'air.

Ainsi, l'opérateur de maintenance venant changer le filtre actif est exposé à des virus ou bactéries désactivées/neutralisées par l'agent actif biodécontaminant disposé à même le filtre actif.

Dans un mode de réalisation, le dispositif de purification d'air comprend une sortie d'air disposée sous le châssis, le châssis étant élevé par rapport à un sol par des roues supportant ledit châssis. Le bruit généré par l'aspiration est réduit. Le dispositif est mobile.

Dans un mode de réalisation, le dispositif de purification d'air comprend un préfiltre disposé en amont du filtre actif dans le sens de circulation de l'air. Le préfiltre empêche le passage des poussières macroscopiques et des insectes.

Dans un mode de réalisation, le dispositif de purification d'air comprend un pressostat disposé entre le filtre actif et l'organe d'amenée d'air et un indicateur d'encrassement des filtres en fonction de la pression détectée par le pressostat. L'indicateur d'encrassement est actif au franchissement d'un plafond de pression, une pression élevée étant caractéristique d'un filtre encrassé. La maintenance est facilitée.

En variante, le pressostat est remplacé par un débitmètre.

Dans un mode de réalisation, l'agent actif biodécontaminant est bactéricide, fongicide et virucide. Les bactéries, virus et levures arrêtés par le filtre actif sont inactivés/neutralisés par ledit filtre actif. Le remplacement du filtre, plus généralement la maintenance, est effectuée en évitant la plupart des risques présents de contamination pour les opérateurs.

Dans un mode de réalisation, l'agent actif biodécontaminant est disposé sous forme pulvérulente à la surface du filtre actif. L'agent actif biodécontaminant recouvre ladite surface du filtre actif, avec de 100 à 150 g/cm² de surface filtrante du filtre actif.

Dans un mode de réalisation, l'agent actif permet d'offre un traitement actif de l'air. Contrairement aux traitements passifs habituels, l'agent actif permet de désactiver les agents pathogènes. En conséquence, la sécurité des opérateurs responsables du changement des filtres est assurée. Le risque de contamination est réduit. Le changement du filtre est facile et ne demande pas l'utilisation d'équipement spécifique.

Dans un mode de réalisation, l'organe d'amenée d'air comprend un ventilateur. Le bruit est réduit.

Dans un mode de réalisation, un mode de réalisation, l'organe d'amenée d'air comprend un variateur faisant varier le débit d'air à traiter selon la taille de la pièce dans laquelle le dispositif de purification est utilisé. La consommation d'énergie électrique est réduite.

Dans un mode de réalisation, l'agent actif biodécontaminant comprend au moins un parmi eugénol, acétate d'eugénol, carvacrol, oligomères proanthocyanidoliques, oleuropéine et hydroxytyrosol.

Dans un mode de réalisation, l'agent actif biodécontaminant comprend au moins 1 % en poids d'eugénol, au moins 0,1 % en poids d'acétate d'eugénol, au moins 0,1 % en poids de carvacrol, au moins 0,01 % en poids d'oligomères proanthocyanidoliques, au moins 1 % en poids d'oleuropéine, et au moins 1 % en poids d'hydroxytyrosol. Les virus sont inactivés rapidement.

Dans un mode de réalisation, l'agent actif biodécontaminant comprend au moins 5 % en poids, d'eugénol, au moins 0,5 % en poids, d'acétate d'eugénol, au moins 0,4 % en poids, de carvacrol, au moins 0,05 % en poids, d'oligomères proanthocyanidoliques, au moins 3 % en poids, d'oleuropéine, et au moins 2 % en poids, d'hydroxytyrosol. Les virus sont inactivés en moins de 30 minutes.

D'autres caractéristiques et avantages de l'invention apparaîtront à l'examen de la description détaillée ci-après, et des dessins annexés, sur lesquels :
La figure 1 illustre de façon schématique une vue de face d'un dispositif selon un aspect de l'invention.
La figure 2 illustre de façon schématique une vue de derrière du dispositif de la figure 1.
La figure 3 illustre de façon schématique une vue de derrière du dispositif de la figure 1.
La figure 4 illustre de façon schématique une vue en coupe dans un plan vertical transversal du dispositif de la figure 1.
La figure 5 illustre de façon schématique une vue en coupe dans un plan horizontal transversal du dispositif de la figure 1.
La figure 6 est une vue de détail de la figure 5.
La figure 7 illustre de façon schématique une vue de face d'un dispositif selon un autre aspect de l'invention.
La figure 8 illustre de façon schématique une vue de derrière du dispositif de la figure 7.
La figure 9 illustre de façon schématique une vue de derrière du dispositif de la figure 1.
La figure 10 illustre de façon schématique une vue en coupe dans un plan vertical transversal du dispositif de la figure 1.

Les dessins annexés pourront non seulement servir à compléter l'invention, mais aussi contribuer à sa définition, le cas échéant.

L'invention a pour but de permettre la filtration d'air et la neutralisation des virus et des bactéries.

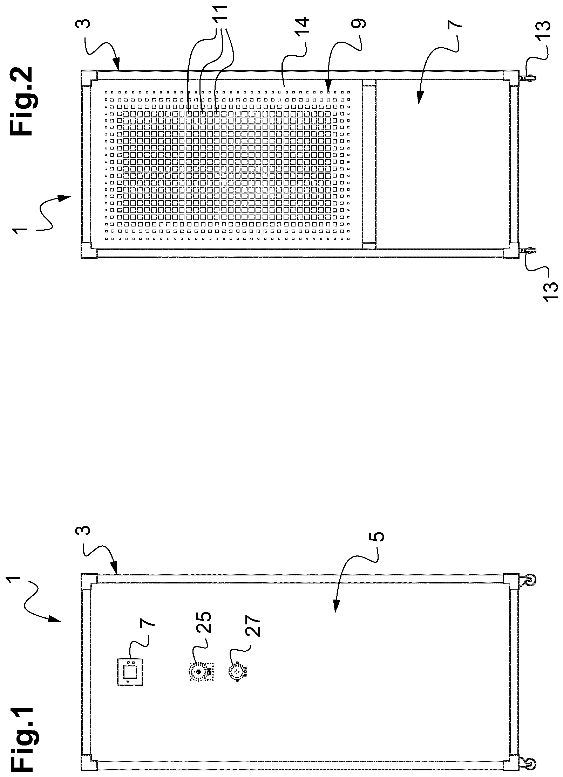

On fait référence aux figures 1 à 3.

Le dispositif de purification d'air 1 comprend un châssis 3. Le châssis 3 est de forme parallélépipédique. En variante, Le châssis 3 peut avoir une forme cylindrique ou conique. Le châssis 3 est fabriqué en aluminium anodisé. Les parois sont fabriquées en acier galvanisé peint.

Ici, le châssis 3 comprend six faces. Une première face avant 5 du châssis 3 comprend un organe de commande 7 pourvu d'une interface homme-machine. L'organe de commande 7 permet de commander le dispositif de purification d'air. Par exemple, l'organe de commande 7 permet de choisir le mode de fonctionnement du dispositif de purification 1.

Le châssis 3 comprend en outre deux faces latérales 7. Ici, chaque face latérale 7 comprend une porte 9. La porte 9 couvre une grande partie haute de la face latérale 7. La porte 9 présente des évidements 11. Les évidements 11 se trouvent sur une grande partie de la porte 9. Les évidements 11 traversent l'épaisseur de la porte. Les évidements 11 sont de dimensions différentes. L'air ambiant est aspiré dans le dispositif de purification 1 via les évidements 11.

Le châssis 3 comprend une face arrière 12. La face arrière 12 peut laisser passage à un câble d'alimentation pour alimenter le dispositif de purification d'air 1.

Le châssis 3 comprend une face supérieure. La face supérieure ferme le châssis 3. Le châssis 3 comprend en outre une face inférieure. La face inférieure comprend des roues 13. Les roues 13 supportent le dispositif de purification d'air 1. Le châssis 3 se trouve élevé par rapport au sol. Les roues 13 permettent de déplacer par roulement le dispositif de purification d'air 1. Ladite face inférieure comprend des évidements, non représentés sur les figures. Les évidements de la face inférieure permettent d'évacuer l'air traité et purifié dans le dispositif de purification d'air.

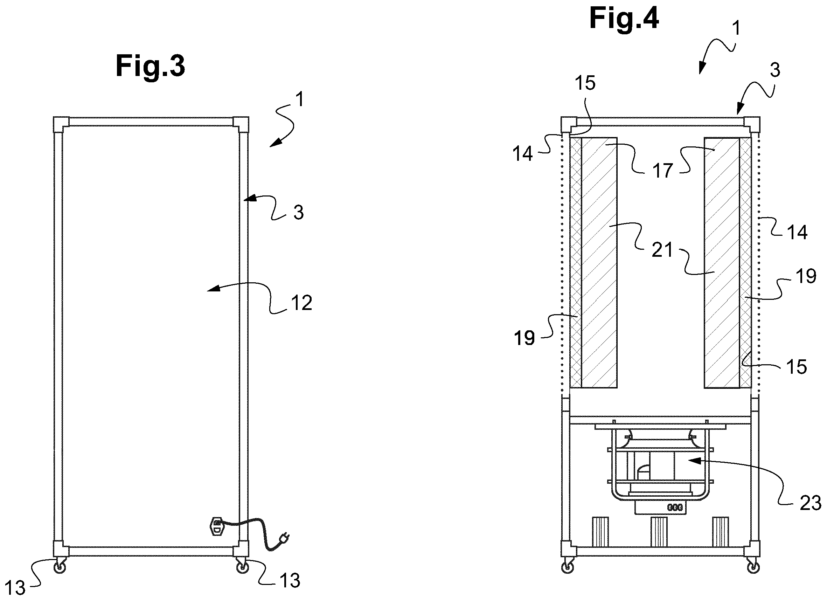

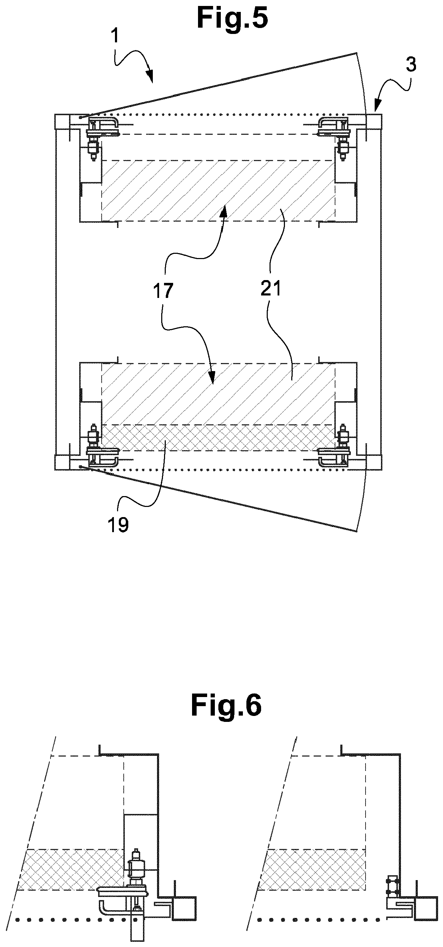

On fait référence aux figures 4 et 5.

La porte 9 présente une face extérieure. La face extérieure 14 est en contact avec l'air ambiant. La porte 9 présente en outre une face intérieure. La face intérieure 15 est en contact avec l'air à l'intérieur du dispositif de purification d'air 1. La face intérieure 15 donne accès à une unité de purification 17. L'unité de purification 17 est de longueur et largeur légèrement inférieures aux longueur et largeur de la porte 9. L'unité de purification 17 couvre tous les évidements 11 présents dans la porte 9.

L'unité de purification 17 est montée sur rail dans le châssis 3. L'unité de purification 17 est amovible pour maintenance, notamment pour le nettoyage. L'unité de purification 17 comprend un premier filtre ou préfiltre 19. Le préfiltre 19 comprend une couche de matériau poreux. Le préfiltre 19 peut être de type, par exemple, ePM1 50% selon la norme ISO 16890 et F7 selon la norme EN 779 2012. Le préfiltre 19 est de dimensions 450x850x50 mm ici. Le préfiltre 19 est entouré par un cadre. Le cadre peut être fabriqué en acier galvanisé à résistance élevée aux chocs. Le cadre entoure un média filtrant. Ledit média filtrant est fabriqué en fibre de verre. Le média filtrant présente une pluralité de plis. Les plis sont séparés par des séparateurs. Les séparateurs maintiennent un espacement uniforme entre chaque pli. La circulation de l'air est ainsi optimale dans et à travers le filtre. Le préfiltre 19 est composé de matériaux incinérables et non corrosifs. La durée de vie du préfiltre 19 est importante.

Un côté du préfiltre 19 contacte en premier l'air aspiré à l'intérieur du dispositif de purification d'air 1 via les évidements 11 de la porte 9. Les poussières macroscopiques et les insectes sont empêchés de passer par le préfiltre 19. Le préfiltre 19 filtre en outre les particules fines. Le préfiltre 19 fait barrière aux particules supérieures à 2,5 µm. Le préfiltre 19 peut fonctionner jusqu'à une température maximale de 70°. Le dispositif de purification d'air 1 est ainsi adapté à fonctionner dans différents endroits.

Un deuxième filtre appelé filtre actif 21 est monté en aval du préfiltre 19. Le montage de l'unité de purification 17 sur rail vient serrer le préfiltre 19 avec le filtre actif 21. Un deuxième côté du préfiltre 19 voit un premier côté du filtre actif 21.

Le filtre actif 21 est ici de dimensions 450x850x117 mm. Le filtre actif 21 comprend deux couches de matériaux poreux. Le filtre 21 est entouré par un cadre. Le cadre est en acier inoxydable. En variante, un cadre fabriqué en tôles de métal galvanisé à joints secs peut être utilisé. Le cadre entoure un média filtrant. Le média filtrant est composé de fibres de verre. Les fibres de verre sont très minces. Préférablement, les fibres de verre sont inférieures à un micron. Les fibres de verre se trouvent assemblées en une feuille à haute densité. Le filtre est configuré en plis en forme de V afin d'obtenir une plus grande surface filtrante. La hauteur du relief des plis est compris entre 30 et 60 mm.

Le filtre actif 21 peut être de type, par exemple, HEPA H14. Ici, la largeur et la longueur du filtre actif 21 sont sensiblement égales à la largeur et la longueur du préfiltre 19, seul l'épaisseur est différente.

Le filtre actif 21 comprend un agent actif biodécontaminant et deux nappes de média filtrant. Ledit agent actif est déposé uniformément sur la surface intérieure des nappes de média filtrant. Préférablement, l'agent actif est sous forme de poudre. Ladite poudre comprend des éléments actifs hydrosolubles. Les éléments actifs hydrosolubles se diffusent sur la surface du filtre actif avec l'augmentation de l'humidité. L'agent actif est ainsi fixé sur le filtre actif 21. Ledit agent actif est pris en sandwich entre les deux nappes de média filtrant.

L'agent actif comprend une pluralité de composants actifs. L'agent actif comprend :
- au moins 1 % en poids, de préférence au moins 5 % en poids, d'eugénol.
- au moins 0,1 % en poids, de préférence au moins 0,5 % en poids, d'acétate d'eugénol.
- au moins 0,1 % en poids, de préférence au moins 0,4 % en poids, de carvacrol.
- au moins 0,01 % en poids, de préférence au moins 0,05 % en poids, d'oligomères proanthocyanidoliques.
- au moins 1 % en poids, de préférence au moins 3 % en poids, d'oleuropéine.
- au moins 1 % en poids, de préférence au moins 2 % en poids, d'hydroxytyrosol.

Le pourcentage en poids de chaque composant est calculé par rapport au poids total de l'agent actif. Les composants actifs dudit agent actif sont actifs sur plusieurs virus et bactéries. C'est-à-dire les composants actifs de l'agent actif permettent de neutraliser une pluralité de virus et bactéries comme le Sars-Cov-2 responsable du Covid-19. La composition de l'agent actif est en outre active sur : Acrodontium Salmoneum, Aspergillus Flavus, Aspergillus fumigatus, Aspergillus Niger, Beauvaria, Campilobacter Jejeuni, Candida Albicans, Cheatomium, Cladosporium Cladosporioides, E-Gergoviae, Human Corona Virus, Klebsiella Pneumonaie, Legionella Pneumophila, Lysteria Monocytogenes, Parinfluenza virus 3, Penicillium Chrysogenum, Phialophora Fastigiata, Pseudonomas Aeruginosa, Rhodotorula et Salmonella Enterica.

La quantité d'agent actif utilisée dépend principalement des dimensions du filtre actif 21 utilisé. Ici, une quantité d'environ 310 grammes est souhaitable pour une filtration et une décontamination optimale. Un grammage surfacique de 100 à 150, par exemple 120, est prévu par cm² de surface développée.

Le filtre actif 21 filtre l'air traversant et empêche le passage des virus et des bactéries. Tandis que l'agent actif biodécontaminant neutralise les virus et les bactéries venant d'être stoppés par le filtre actif 21. L'agent actif biodécontaminant est bactéricide, fongicide et virucide. Ainsi la combinaison du filtre actif 21 et de l'agent actif permet un traitement actif de l'air traversant l'unité de purification 17, contrairement aux traitements passifs habituels.

Le dispositif de purification d'air 1 comprend un organe d'amenée 23 d'air ambiant. Ledit organe d'amenée 23 d'air est ici disposé en aval du filtre actif 21 dans le sens de la circulation de l'air. L'organe d'amenée 23 d'air comprend préférablement un ventilateur. Ledit organe d'amenée 23 d'air est disposé en dessous de l'unité de purification 17. Une première partie stable de l'organe d'amenée d'air 23 est solidaire au châssis 3. Une deuxième partie de l'organe d'amenée 23 d'air est tournante. L'organe d'amenée 23 comprend en outre un variateur. Le variateur fait varier le débit d'air amené par l'organe d'amenée 23. L'organe d'amenée 23 d'air fait circuler l'air ambiant. La partie tournante de l'organe d'amenée 23 d'air ramène l'air ambiant de l'extérieur via les évidements 11. Avant que l'air ambiant entre à l'intérieur du châssis 3, l'unité de purification 17 le filtre et le neutralise. Le préfiltre 19 filtre les poussières macroscopiques, les insectes et les particules fines. Puis, Le filtre actif 21 filtre les virus et les bactéries. Et l'agent actif neutralise lesdits virus et bactéries emprisonnés par le filtre actif 21. L'air purifié est ensuite évacué à l'extérieur via les évidements de la face inférieure du châssis 3.

Le dispositif de purification d'air 1 comprend un pressostat 25. Le pressostat 25 est disposé entre le filtre actif 21 et l'organe d'amenée 23 d'air. Le dispositif de purification d'air 1 comprend en outre un transmetteur de pression 27. Le transmetteur de pression 27 est disposé entre le filtre actif 21 et l'organe d'amenée 23 d'air.

On fait référence à la figure 6.

Le mécanisme d'ouverture et de fermeture de la porte 9 comprend des taquets à ressort mâle et femelle, ainsi qu'un loquet quart de tour avec serrure de sécurité. Un contacteur de sécurité coupe l'alimentation électrique dés ouverture de la porte de maintenance.

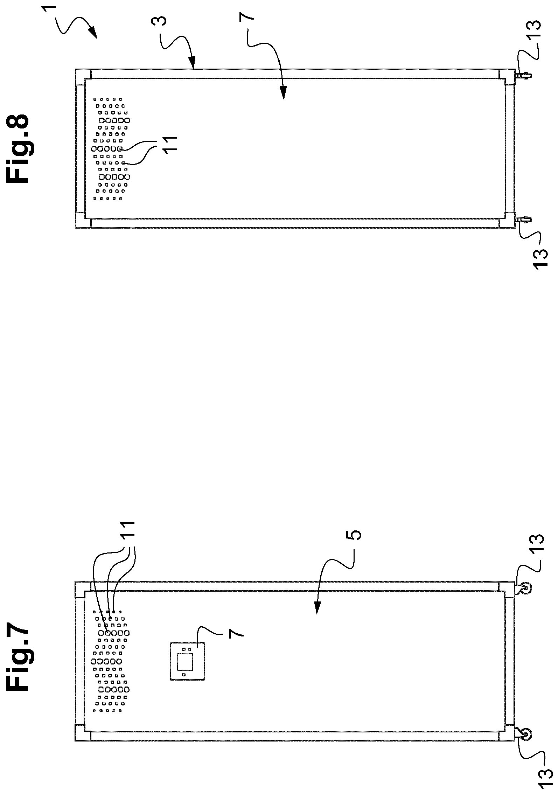

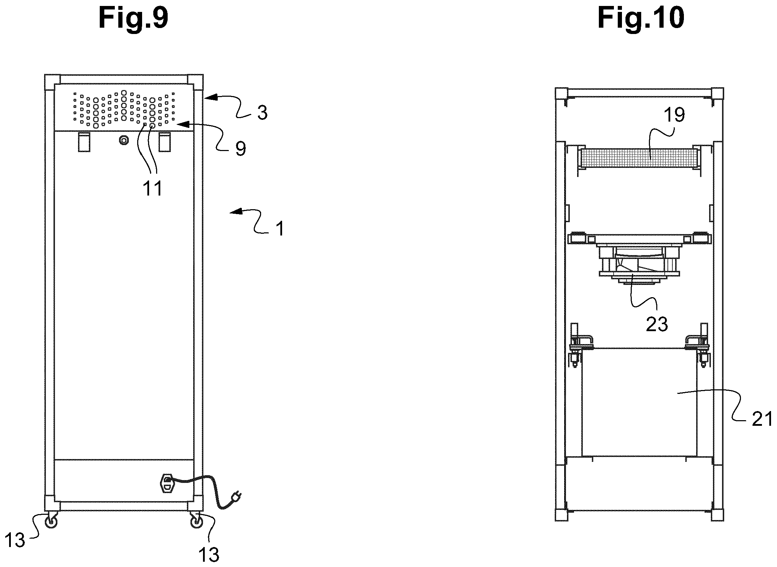

On fait référence aux figures 7 à 10.

Le châssis 3 du dispositif de purification d'air est semblable à celui du mode réalisation des figures 1 à 6, à ceci près que, la première face avant 5 du châssis 3 comprend en outre les évidements 11. Les évidements 11 couvrent moins de surface que dans le mode de réalisation précédent. Les évidements 11 sont disposés sur une partie haute de la face 5. Ladite partie haute présente une forme rectangulaire. Ladite partie haute couvre, par exemple, 10% de la superficie de la face avant 5.

Ici, chaque face latérale 7 du châssis 3 comprend les évidements 11. Les évidements 11 des faces latérales 7 sont disposés sensiblement de la même façon que les évidements de la face avant 5.

Ici, la face arrière 7 du châssis 3 comprend une porte 9. La porte 9 couvre une partie haute de la face arrière 12. La porte 9 couvre ici moins de superficie de la face arrière 12. La porte 9 présente les évidements 11. Les évidements 11 de la face arrière 12 sont disposés sensiblement de la même façon que les évidements de la face avant 7. La face arrière 11 peut aussi laisser passage à un câble d'alimentation pour alimenter le dispositif de purification d'air 1.

Comme dans le mode de réalisation des figures 1 à 6, le châssis 3 comprend la face supérieure fermant le châssis 3 et une face inférieure comprenant des roues 13. Les roues 13 supportent le dispositif de purification d'air 1. Le châssis 3 se trouve élevé par rapport au sol.

Les parties hautes des faces avant 5, latérales 7 et arrière 12 présentant les évidements 11 sont plus minces que le reste des faces avant 5, latérales 7 et arrière 12. L'épaisseur faible des extrémités hautes facilite la circulation de l'air.

La face extérieure est en contact avec l'air ambiant. La face intérieure est en contact avec l'air à l'intérieur du dispositif de purification d'air 1. La face intérieure est en regard d'un premier composant d'une unité de purification 17. L'unité de purification ici est composé de deux composants disposés séparés l'un de l'autre.

Comme le premier mode de réalisation, ledit premier composant est le préfiltre 19. Le préfiltre 19 est semblable au préfiltre utilisé pour le mode de réalisation des figures 1 à 6 à ceci près que, le préfiltre 19 est de dimensions 305x305x48 mm. Des dimensions différentes pour autres modes de réalisation sont prévues. Le côté du préfiltre 19 contacte en premier l'air aspiré à l'intérieur du dispositif de purification d'air 1 via les évidements 11. Les poussières macroscopiques, les insectes et les particules fines sont empêchés de passer par le préfiltre 19. Ainsi, l'air préfiltré passe sort du deuxième côté du préfiltre 19. Le préfiltre 19 est disposé parallèle à la face inférieure du dispositif de purification d'air 1 destinée à évacuer l'air traité. Le préfiltre est monté seul sur rail dans le châssis 3. Le préfiltre est amovible pour maintenance, notamment pour le nettoyage.

Comme dans le mode de réalisation des figures 1 à 6, le dispositif de purification d'air 1 comprend l'organe d'amenée 23 d'air ambiant. Ledit organe d'amenée 23 d'air est ici disposé en aval du préfiltre 19 et en amont du deuxième composant de l'unité de purification 17.

Ledit deuxième composant de l'unité de purification est ledit filtre actif 21. Le filtre actif 21 est semblable au filtre actif utilisé pour le mode de réalisation des figures 1 à 6 à ceci près que, le filtre actif 21 est de dimensions 305x305x292 mm ici. Des dimensions différentes pour autres modes de réalisation sont prévues. Ici, L'épaisseur du filtre actif 21 est plus importante que celle du premier mode de réalisation. L'épaisseur du filtre actif 21 ici est environ trois fois plus grande que celle du premier mode de réalisation. Le filtre est de gamme HEPA H14. Un tel filtre à très haute efficacité permet de bloquer des particules jusqu'à 0,05 µm. Le premier étage de filtration protège le motoventilateur contre les poussières. Le deuxième étage de filtration effectue la purification d'air en cumulant la poudre d'agent actif et l'efficacité H14. Le filtre actif 21 est disposé parallèle à la face inférieure du dispositif de purification d'air 1 destinée à évacuer l'air traité. Le filtre actif 21 est monté seul sur rail dans le châssis 3.

Comme dans le mode de réalisation des figures 1 à 6, le filtre actif 21 comprend l'agent actif biodécontaminant qui neutralise les virus et les bactéries stoppés par le média filtrant. Ainsi, Les deux composants de l'unité de purification 17 se trouvent séparés ici en deux étages de filtration : Etage 1 avec Filtre F7 pour les poussières et étage 2 avec filtre H14 et agent actif contre les bactéries. L'organe d'amenée d'air 23 fait circuler l'air ambiant. L'organe d'amenée d'air 23 ramène l'air ambiant de l'extérieur via les évidements 11. Avant que l'air ambiant passe par le ventilateur de l'organe d'amenée d'air 23, Le préfiltre 19 filtre les poussières macroscopiques, les insectes et les particules fines. Puis, L'air est aspiré par l'organe d'amenée 23. Ensuite, L'air passe par le filtre actif 21. Le filtre actif 21 filtre les virus et les bactéries. Et l'agent actif neutralise lesdits virus et bactéries emprisonnés par le filtre actif 21. L'air filtré et décontaminé est ensuite évacué à l'extérieur via les évidements de la face inférieure du châssis 3. Un agent de maintenance peut changer le filtre sans risque notable de contamination et sans utilisation d'équipements spécifiques de sécurité.

## Revendications

1. Dispositif de purification d'air (1), comprenant un châssis (3) déplaçable, un organe d'amenée d'air (23) ambiant dans le dispositif, un filtre actif (21), un agent actif biodécontaminant déposé à la surface du filtre actif, l'organe d'amenée d'air (23) étant disposé en aval ou en amont du filtre actif (21) dans le sens de circulation de l'air.

2. Dispositif selon la revendication 1, comprenant une sortie d'air disposée sous le châssis, le châssis étant élevé par rapport à un sol par des roues (13) supportant ledit châssis (3).

3. Dispositif selon la revendication 1 ou 2, comprenant un préfiltre (19) disposé en amont du filtre actif (21) dans le sens de circulation de l'air.

4. Dispositif selon l'une des revendications précédentes, comprenant un pressostat (25) disposé entre le filtre actif (21) et l'organe d'amenée d'air (23) et un indicateur d'encrassement des filtres en fonction de la pression détectée par le pressostat (25).

5. Dispositif selon l'une des revendications précédentes, dans lequel l'agent actif biodécontaminant est bactéricide, fongicide et virucide.

6. Dispositif selon l'une des revendications précédentes, dans lequel l'agent actif biodécontaminant est disposé sous forme pulvérulente à la surface du filtre actif (21).

7. Dispositif selon l'une des revendications précédentes, dans lequel l'organe d'amenée d'air (23) comprend un ventilateur.

8. Dispositif selon l'une des revendications précédentes, dans lequel l'organe d'amenée d'air (23) comprend un variateur faisant varier le débit d'air à traiter.

9. Dispositif selon l'une des revendications précédentes, dans lequel l'agent actif biodécontaminant comprend :
- au moins 1 % en poids d'eugénol,
- au moins 0,1 % en poids d'acétate d'eugénol,
- au moins 0,1 % en poids de carvacrol,
- au moins 0,01 % en poids d'oligomères proanthocyanidoliques,
- au moins 1 % en poids d'oleuropéine.
- au moins 1 % en poids d'hydroxytyrosol.

10. Dispositif selon la revendication 9, comprenant
- au moins 5 % en poids, d'eugénol,
- au moins 0,5 % en poids, d'acétate d'eugénol,
- au moins 0,4 % en poids, de carvacrol,
- au moins 0,05 % en poids, d'oligomères proanthocyanidoliques,
- au moins 3 % en poids, d'oleuropéine,
- au moins 2 % en poids, d'hydroxytyrosol.
